# EUROPEAN PATENT SPECIFICATION

(11) **EP 3 182 120 B1**
(45) Date of publication and mention of the grant of the patent: **22.02.2023**
(21) Application number: 16203715.4
(22) Date of filing: 13.12.2016
(51) Int. Cl.: G01N 33/28

(54) **A GC/GC-MS BASED METHOD FOR THE ESTIMATION OF TRACE LEVEL CONJUGATED DIENES IN GASOLINE RANGE PRODUCTS**
GC/GC-MS-BASIERTES VERFAHREN ZUR SCHÄTZUNG VON KONJUGIERTEN DIENEN IM SPURENBEREICH IN BENZINBEREICHSPRODUKTEN
PROCÉDÉ À BASE DE GC/CG-SM D'ESTIMATION DE NIVEAU DE TRACE DE DIÈNES CONJUGUÉS DANS DES PRODUITS ENTRANT DANS LA GAMME DES ESSENCES

(30) Priority: 14.12.2015 IN 4690MU2015
(43) Date of publication of application: 21.06.2017
(73) Proprietor: INDIAN OIL CORPORATION Ltd., Mumbai 400 051 (IN)
(72) Inventor: CHOPRA, Anju, 121007 Haryana (IN); KUMAR, Balivada Ravi, 121007 Haryana (IN); SINGH, Bhawna, 304022 Rajastan (IN); SINGH, Dheer, 121007 Haryana (IN); KAGDIYAL, Vivekanand, 121007 Haryana (IN); GUPTA, Anurag Ateet, 121007 Haryana (IN)
(74) Representative: Herrero & Asociados, S.L.

(56) References cited:
- US-B2- 6 869 803
- ANDRADE D F ET AL: "Comparison of UOP-326, voltammetric and gas chromatographic/mass spectrometric methods for the determination of conjugated dienes in Brazilian Naphtha", FUEL, IPC SCIENCE AND TECHNOLOGY PRESS, GUILDFORD, GB, vol. 85, no. 7-8, 1 May 2006 (2006-05-01), pages 1024-1031, XP028064375, ISSN: 0016-2361, DOI: 10.1016/J.FUEL.2005.10.007 [retrieved on 2006-05-01]
- KATHLEEN E. EDWARDS ET AL: "Quantitative Analysis of Conjugated Dienes in Hydrocarbon Feeds and Products", ENERGY & FUELS, vol. 19, no. 5, 1 September 2005 (2005-09-01), pages 2034-2040, XP055153931, ISSN: 0887-0624, DOI: 10.1021/ef0500540
- ANIL YADAV ET AL: "HPLC Method for Monitoring the Conjugated Dienes and Olefins in FCC, Coker Gasolines, and their Hydrogenated Products", JOURNAL OF LIQUID CHROMATOGRAPHY AND RELATED TECHNOLOGIES, vol. 38, no. 8, 9 May 2015 (2015-05-09), pages 840-846, XP055358102, US ISSN: 1082-6076, DOI: 10.1080/10826076.2014.968663

## Description

### FIELD OF THE INVENTION

The present invention is related to a method for the estimation of conjugated dienes in gasoline range samples by Gas Chromatography coupled with Mass Spectrometry. In particular, the method is used to estimate C₅ to Cs di-olefins which form a major part of the gasoline composition.

### BACKGROUND OF THE INVENTION:

Conjugated dienes are a major class of compounds in hydrocarbon systems that are responsible for deposit formation in refinery conversion units such as hydrocracking and hydro-conversion units and fractionators and heat exchangers. They are also responsible for deposit formation in automobile engines. The information is critical for assessing the potential of forming deposits in a hydrocarbon system. Knowledge of the concentration and types of conjugated dienes provides guidance to the refinery on the necessity of pre-treating the refinery feed, such as installing a diolefin saturator prior to processing in order to prevent fouling during processing. In fuel applications, the information may be used to determine, e.g. if and how much a naphtha can be blended into fuel or if the naphtha needs to be hydrotreated etc.

There are no reliable established methods to identify and quantify conjugated dienes, acyclic and cyclic, in hydrocarbon systems. The existing method for determining dienes in petroleum distillates is UOP-326, which is by definition, the number of grams of iodine equivalent to the amount of maleic anhydride that reacts with 100g of the sample (on the basis of 2 atoms of iodine per mole of maleic anhydride). As the method says, this determination is somewhat empirical and is very cumbersome and lengthy.

GC/FID (Flame ionization detector) can be used to quantify small conjugated dienes (Less than C₆) in low boiling hydrocarbon systems. The identifications were made based on retention times and mass spectral library matches. The method cannot identify/quantify large conjugated dienes (Greater than C₆) due to the low concentrations of the analytes, severe overlaps with other hydrocarbon components and very similar mass spectra between conjugated and non-conjugated dienes.

Efforts have been made in the past to estimate conjugated dienes in gasoline range products by various Analytical Techniques such as NMR (Nuclear magnetic resonance), Near IR (Infrared), HPLC (High performance Liquid chromatography), SFC (Super critical fluid chromatography) and GC-MS. Bromination has been widely used to determine olefin content (including conjugated dienes) in hydrocarbons. It cannot differentiate conjugated dienes from other olefins. Aromatics, especially phenols, can interfere with the analysis. The MAV test is a semiquantitative method developed by UOP and uses maleic anhydride as a derivatization agent. The reaction is not selective and cannot proceed quantitatively. Therefore, only a relative number can be obtained. In addition, it cannot provide molecular identifications of different conjugated diene types required for refinery guidance.

Another approach by Exxon Mobil has reported a method by which quantification has been done by their derivatization with MTAD (4-methyl-1,2,4,-triazoline-3,5-dione).

US6869803 discloses method to quantitatively analyze conjugated dienes in hydrocarbon feeds and products as an indicator of fouling potential. It discloses the method to quantify the conjugated dienes in a feedstream including the steps of dissolving 4-methyl-1,2,4-trazoline-3,5-dione (MTAD) in said feedstream, and determining the molar concentration of said conjugated dienes and/or the carbon number distribution of said conjugated dienes by GC/MS and GC/NCD (Nitrogen Chemiluminescence Detection). One of the main disadvantages of this prior art is that, addition to conjugated dienes, other compounds such as aromatics having conjugated double bonds also tend to react with MTAD, resulting in erroneous results.

Furthermore, quantitative determination of hydrocarbon species including diolefins in a complex mixture of hydrocarbons by Gas Chromatography/Mass Spectrometry is known in the art like it is known in US7153694. However, the method disclosed in US'694 use Townsend Discharge Nitric Oxide Chemical Ionization Gas Chromatography/Mass Spectrometry. The main shortcoming of this prior art is that this method does not claim to identify and quantify the individual conjugated dienes. Its calibrates all the classes using a carbon number (such as C₆ &

C₇) in a particular functional group and the results are reported as consolidated carbon number. Further publications on this subject include ANDRADE D F ET AL: "Comparison of UOP-326, voltammetric and gas chromatographic/mass spectrometric methods for the determination of conjugated dienes in Brazilian Naphtha",FUEL, IPC SCIENCE AND TECHNOLOGY PRESS, GUILD FORD, GB, vol. 85, no. 7-8, 1 May 2006 (2006-05-01), pages 1024-1031 and KATHLEEN E. EDWARDS ET AL: "Quantitative Analysis of Conjugated Dienes in Hydrocarbon Feeds and Products",ENERGY & FUELS, vol. 19, no. 5, 1 September 2005 (2005-09-01), pages 2034-2040.

The majority of the methods known in prior art teaches the total diene content by GC coupled with NCD (Nitrogen chemiluminescence detector) or Mass Detectors. However, the need of the refinery is to identify particular dienes which are getting hydrogenated during the Selective Hydrogenation Process (SHP) before the desulfurization of the fuel. Therefore, there is a need to develop new method that involves speciation of olefins with their exact quantitative estimation by GC / GC-MS. GC-MS provides the typical fragmentation pattern of a diene which distinguishes itself from the mono-olefins. There is a need to develop a method wherein the Selective Hydrogenation Unit does not affect the mono-olefins in order to maintain the gasoline composition and the method is able to estimate C₅ to Cs di-olefins which form a major part of the gasoline composition.

### Objective of the invention:

It is the primary objective of this invention to provide a method to quantify the conjugated dienes in gasoline.

It is further objective of the present invention to provide a method which is able to separate and identify and estimate around 25 different conjugated dienes present in gasoline.

It is further objective of the present invention to provide a method that can estimate C₅ to Cs di-olefins which form a major part of the gasoline composition.

### Summary of the invention:

The present invention provides a method that detects, identifies and quantifies conjugated dienes in various hydrocarbon matrices, such as steam cracked naphtha, catalytic cracked naphtha, coker naphthas, and other petroleum distillates.

The present invention relates to a method of speciation and quantification of conjugated dienes in a feed or a product stream by a Gas Chromatography (GC) wherein said Gas Chromatorgraphy (GC) is coupled with Mass Spectrometry.

The present invention further relates to a method of determining the amount of conjugated dienes in a selective hydrogenation process (SHP) wherein said method comprises of
a) passing a portion of a feed or a product stream to be processed by the SHP, through a GC column having a length ranging from 140-170 m ;
b) determining concentration of each conjugated dienes by a mass spectrometer;
c) performing the SHP on the feed or the product stream to obtain a SHP product;
d) passing a portion of the SHP product through the GC column having a length ranging from 140-170 m;
e) determining concentration of each conjugated dienes by the mass spectrometer; and
f) comparing the concentration of the conjugated dienes of step (b) and (e) to determine the amount of hydrogenation of conjugated dienes in the SHP.

The present invention further relates to a method which is used to separate and identify and estimate about 25 different conjugated dienes selected from the group comprising of 2,4-pentadiene, 1,3-pentadiene (Z), 1,3-pentadiene (E), 2-methyl-1,3-pentadine (E), 2-methyl-1,3-pentadiene (Z), 3-methyl-1,3-pentadiene (E), 3-methyl-1,3-pentadiene (Z), 2,4-hexadiene (E,E), 2,4-hexadiene (Z,Z), 2,4-hexadiene (E,Z), 2,4-hexadiene (Z,E), 1,3-hexadiene (E), 1,3-hexadiene (Z), 1-methyl-1,3-cyclopentadiene (E), 1-methyl-1,3-cyclopentadiene (Z), 2-methyl-1,3-pentadiene, 4-methyl-1,3-pentadiene, 2-3-dimethyl-1,3-pentadiene, 3,3-dimethyl-1,3-pentadiene, 5-5-dimethyl-1,3-hexadiene, 1,3,5-heptatriene, 2,3,4-trimethyl-1,3-pentadiene, 1-methyl-cyclohexa-1,3-diene, 2-methyl-cyclohexa-1,3-diene and 1,3,5-trimethyl-1,3-pentadiene.

The present invention furthermore relates to a method to quantify conjugated dienes in a Selective Hydrogenation Unit (SHU) feed and a product sample wherein said method comprises of:
a) setting up a Scion 436 GC with Bruker SQ mass detector (Quadrupole Mass) with a 150 meter CP SIL 5 CB column non-polar column (150 m x 0.23mm x 0.5mm);
b) tuning mass spectrometer for unit resolution in a mass range of 1 to 1000 Da using Per-fluoro tertiary butyl amine (PFTBA) as a reference for calibration of masses;
c) maintaining the Bruker SQ mass detector at a high vacuum of 10⁻⁶ bar and the temperature of the filament and transfer line at 260°C;
d) setting up an oven program for separation of conjugated dienes in a SHU feed and a product matrix;
e) injecting a chloroform solution of a standard (s);
f) calculating response factor of the standard (s) of step (e) and calculating an average response factor and selecting one conjugated diene as a representative diene for calibration;
g) preparing a calibration curve in the concentration range 200 ppm to 1% (w/w) against internal standard;
h) analyzing actual SHU feed and product samples containing internal standard and determining the peak area of conjugated dienes identified with the help of NIST mass spectral library; and
i) calculating the concentration of total conjugated dienes and estimating the concentration against calibration curve of the conjugated diene of step (f).

### Diagrams:

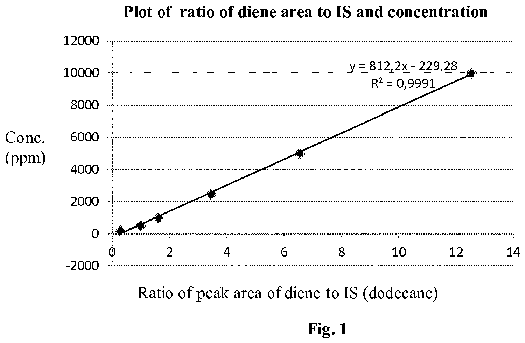

**Fig. 1****:** Calibration Curve for the estimation of conjugated dienes

### Descriptionoftheinvention:

The tables have been represented where appropriate by conventional representations, showing only those specific details that are pertinent to understanding the embodiments of the present invention so as not to obscure the disclosure with details that will be readily apparent to those of ordinary skill in the art having benefit of the description herein.

The following description is of exemplary embodiments only and is not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention.

It is known that the Gasoline generated from the cracking process contains conjugated dienes in the carbon range Cs to Cs which are highly prone to polymerization process and cause refining units plugging and shut down of the reactor. These dienes are removed by selective hydrogenation process (SHP) which brings down the concentration of conjugated dienes from 0.2 to 2% in the feed samples to ppm level in the SHU product.

The existing method for determining dienes in petroleum distillates is UOP-326, which is somewhat empirical and is very cumbersome and lengthy method whereas the GC based method is a quick method and is much more accurate since the dienes are identified by injecting dienes standards procured from M/s Sigma Aldrich. The inventors of the present invention have explored the GC based method for determining the dienes by using fragmentation patterns from mass spectrometer NIST library which are estimated against pure dienes standard. By this method, the detection limit of 100ppm has been attained. The total conjugated diene concentration can also be estimated by programme which elutes the high boiling adducts as total C₅, C₆, C₇ and C₈ dienes. A correlation was drawn between the results obtained by UOP-326 method and the total conjugated dienes concentration estimated by GC-MS of the present invention. The correlation coefficient was found to be 0.989.

Accordingly, the main embodiment of the present invention relates to a method of quantification of conjugated dienes in a feed or a product stream by a Gas Chromatography (GC) wherein said Gas Chromatorgraphy (GC) is coupled with Mass Spectrometry in a selective hydrogenation process (SHP) wherein said method comprises of:
a) passing a portion of a feed or a product stream to be processed by the SHP, through a GC column having a length ranging from 140-170 m;
b) determining concentration of each conjugated dienes by a mass spectrometer;
c) performing the SHP on the feed or the product stream to obtain a SHP product;
d) passing a portion of the SHP product through the GC column having a length ranging from 140-170 m;
e) determining concentration of each conjugated dienes by the mass spectrometer; and
f) comparing the concentration of the conjugated dienes of step (b) and (e) to determine the amount of hydrogenation of conjugated dienes in the SHP.

In further embodiment, the method involves passing a portion of a feed or a product stream to be processed by the SHP, through a GC column and determining concentration of each and/ or total conjugated dienes by a mass spectrometer.

In another embodiment, the feed or the product stream passed through the GC column is gasoline.

In further embodiment, the conjugated dienes contains carbon in the range of C₅ to Cs wherein the conjugated dienes are cyclic, open chain, branched chain, or a mixture thereof.

Preferably, the conjugated dienes that are detected by the process of the present invention include the group comprising 2,4-pentadiene, 1,3-pentadiene (Z), 1,3-pentadiene (E), 2-methyl-1,3-pentadiene (E), 2-methyl-1,3-pentadiene (Z), 3-methyl-1,3-pentadiene (E), 3-methyl-1,3-pentadiene (Z), 2,4-Hexadiene (E,E), 2,4-Hexadiene (Z,Z), 2,4-Hexadiene (E,Z), 2,4-Hexadiene (Z,E), 1,3-Hexadiene (E), 1,3-Hexadiene (Z), 1-methyl-1,3-cyclopentadiene(E), 1-methyl-1,3-cyclopentadiene (Z), 2-methyl-1,3-pentadiene, 4-methyl-1,3-pentadiene, 2,3-dimethyl-1,3-pentadiene, 3,3-dimethyl-1,3-pentadiene, 5,5-dimethyl-1,3-hexadiene, 1,3,5-heptatriene, 2,3,4-trimethyl-1,3-pentadiene, 1-methyl-cyclohexa-1,3-diene, 2-methyl-cyclohexa-1,3-diene, 1,3,5-trimethyl-1,3-pentadiene.

In a preferred embodiment, the GC column is non-polar in nature. Preferably, the gas chromatography column for the quantification and speciation of dienes operate at a temperature range of 60-360°C. Advantageously, the use of 150m GC column provides sufficient resolution between conjugated dienes and other class of olefins.

In a preferred embodiment, the feed or product stream is gasoline. Preferably, the method of the present invention proves to be efficient enough to estimate conjugated dienes in gasoline before and after the selective hydrogenation process.

Moreover, the method of the present invention can be used to determine the low amounts (even the trace amounts) of conjugated dienes to the extent of ppm.

In one another preferred embodiment, the mass spectrometer is employed with a quadrupole mass detector wherein the mass detector is tuned for unit resolution in mass range of 1 to 1000 Da by using per-fluoro tertiary butyl amine as reference for calibration of masses. Further, the mass detector is operated at a vacuum of about 10⁻⁶ bar and temperature of filament and transfer line is 240-280°C. In another preferred embodiment, the temperature of filament and transfer line is 260°C.

In further embodiment, the present invention provides a method to quantify the conjugated dienes in a SHU feed and product sample wherein said method comprises of:
a) setting up a Scion 436 GC with Bruker SQ mass detector (Quadrupole Mass) with a 150 meter CP SIL 5 CB column non-polar column (150 m x 0.23mm x 0.5mm);
b) tuning mass spectrometer for unit resolution in a mass range of 1 to 1000 Da using Per-fluoro tertiary butyl amine (PFTBA) as a reference for calibration of masses;
c) maintaining the Bruker SQ mass detector at a high vacuum of 10⁻⁶ bar and the temperature of the filament and transfer line at 260°C;
d) setting up an oven program for separation of conjugated dienes in a SHU feed and a product matrix;
e) injecting a chloroform solution of a standard (s);
f) calculating response factor of the standard (s) of step (e) and calculating an average response factor and selecting one conjugated diene as a representative diene for calibration;
g) preparing a calibration curve in the concentration range 200 ppm to 1% (w/w) against internal standard;
h) analyzing actual SHU feed and product samples containing internal standard and determining the peak area of conjugated dienes identified with the help of NIST mass spectral library; and
i) calculating the concentration of total conjugated dienes and estimating the concentration against calibration curve of the conjugated diene of step (f).

In preferred embodiment the present invention provides a method to quantify the conjugated dienes in a SHU feed and product sample wherein said method comprises of:
a) setting up a Scion 436 GC with Bruker SQ mass detector (Quadrupole Mass) with a 150 meter CP SIL 5 CB column non-polar column (150 m x 0.23mm x 0.5mm);
b) tuning mass spectrometer for unit resolution in the mass range of 1 to 1000 Da using Per-fluoro tertiary butyl amine (PFTBA) as reference for calibration of masses;
c) maintaining the source of the Bruker SQ mass detector at a high vacuum of 10⁻⁶ bar and the temperature of the filament and transfer line at 260°C;
d) setting up the following oven program for the separation of conjugated dienes in the SHU feed and product matrix:

| | |
|---|---|
| 35°C (10minhold) -------0.5°C/min---------> | 60°C |
| 60°C (5min hold) -------1.0°C/min---------> | 150°C |
| 150°C (5min hold) -------70°C/min ---------> | 250°C (30 min); |

e) injecting a chloroform solution of the standards in the carbon range Cs to Cs viz., t-1,3-pentadiene, 3-methyl-1,3-pentadiene, 2,4-dimethyl-1,3-pentadiene, 1,3-hexadiene, 2,4-hexadiene, 1,3-cyclohexadiene, 1,3-cyclooctadiene (1% each);
f) calculating the response factor of above seven conjugated dienes and calculating an average response factor and selecting one conjugated diene, viz., 1, 3-cyclooctadiene as a representative diene for calibration;
g) preparing a calibration curve for 1,3-cyclooctadiene in the concentration range 200 ppm to 1% (w/w) against dodecane as the internal standard;
h) analyzing actual SHU feed and product samples containing dodecane (IS) and determining the peak area of conjugated dienes identified with the help of NIST (National Institute of Standards and Technology) mass spectral library (2011); and
i) calculating the concentration of total conjugated dienes (by adding up the peak area of individual dienes) and estimating the concentration against calibration curve of 1,3-cyclooctadiene.

The present invention provides the method which can be used to separate and identify and estimate up to 25 different conjugated dienes present in gasoline. The said 25 different conjugated dienes are selected from 2,4-pentadiene, 1,3-pentadiene (Z), 1,3-pentadiene (E), 2-methyl-1,3-pentadine (E), 2-methyl-1,3-pentadiene (Z), 3-methyl-1,3-pentadiene (E), 3-methyl-1,3-pentadiene (Z), 2,4-hexadiene (E,E), 2,4-hexadiene (Z,Z), 2,4-hexadiene (E,Z), 2,4-hexadiene (Z,E), 1,3-hexadiene (E), 1,3-hexadiene (Z), 1-methyl-1,3-cyclopentadiene (E), 1-methyl-1,3-cyclopentadiene (Z), 2-methyl-1,3-pentadiene, 4-methyl-1,3-pentadiene, 2-3-diemethyl-1,3-pentadiene, 3,3-dimethyl-1,3-pentadiene, 5-5-dimethyl-1,3-hexadiene, 1,3,5-heptatriene, 2,3,4-trimethyl-1,3-pentadiene, 1-methyl-cyclohexa-1,3-diene, 2-methyl-cyclohexa-1,3-diene and 1,3,5-trimethyl-1,3-pentadiene.

The method is used to estimate conjugated dienes in gasoline range products before and after the selective hydrogenation process in the refineries. The process is used to remove conjugated dienes from the stream before the DHDS (Diesel Hydrodesulfurization) process for the production of Euro-IV / Euro-V gasoline.

Moreover, the GC based method of the present invention is a quick method and is much more accurate and it detects typical fragmentation pattern of a diene which distinguishes itself from the mono-olefins.

The invention will now be explained with the help of following examples. However, the scope of the invention should not be limited to these examples as the person skilled in the art can easily vary the proportion of the ingredients and combinations.

### Experiments

The conjugated dienes speciation has been carried out on a 150m CPSIL PONA CB non-polar GC column from M/s Zebron (ZB-1) having a temperature range of 60 to 360°C having dimensions 150m x 0.25mm × 1.0µm. The earlier works have been attempted on 30 to 60 m GC columns which do not provide the sufficient resolution between conjugated dienes and other classes of olefins. The response factors of 8 different types of conjugated dienes have been estimated by analyzing individual diene standards from M/s Sigma Aldrich (99.9% pure or higher). The standard test method for the component speciation in gasoline is as per ASTM D 6730 GC based method which gives the PIONA analysis at each carbon number. The method separates mono-olefins up to a maximum concentration of 25% max but does not mention about di-olefins. The coker gasoline sample from IOC Refinery is analysed as per ASTM D 6730 method even under cryo conditions with oven programme starting from 5°C. But the diolefins could not be resolved. In order the increase the column efficiency, by increasing the length of the column, the separation of diolefins was attempted using commercially available pure diolefin standards procured from M/s Sigma Aldrich. A 150 metre CPSIL PONA CB column (150 m x 0.25mm) from M/s Zebron is used to carry out the analysis. For calibration purpose, the standards procured for diolefins ranged from Cs to Cs diolefins, viz., isoprene, t-1,3-pentadiene, 3-methyl-1,3-pentadiene, 2,4-dimethyl-1,3-pentadiene, 1,3-hexadiene, 2,4-hexadiene, 1,3-cyclohexadiene, 1,3-octadiene, 1,3-cyclooctadiene etc. The gasoline sample doped with the diolefins is analysed on the fabricated 150 metre column using a 191 metre oven program and it is found that almost all the dienes are resolved and could be identified using NIST mass spectral library.

### Calibration of conjugated dienes

In order to establish the differences in the response factors of various dienes, 1% seven conjugated dienes of varied nature are analysed by the developed method. Some of the dienes, viz., 2,4-hexadiene are the mixture of isomers. By adding the areas of all the isomers of a diene, it is observed that irrespective of the nature of diene (cyclic, open chain or branched), not much variation is there in the response factor. Therefore, octadiene is used for calibration in the concentration range of 200ppm to 1% against dodecane as the internal standard. Dodecane gets well separated from the gasoline matrix and there is no overlapping of the hydrocarbon with any of the dienes.

### Experimental Data:

**Table 1:**

| **Sample** | **Diene Valve by UOP-326** | **Diene Concentration by GC-MS (wt%)(against cyclo-octadiene)** |
|---|---|---|
| | **(D)** | |
| | | **IS : Dodecane** |
| Coker Gasoline ( Digboi) | 1.42 | 0.38% (0.45)* |
| SHU product (PR) | 0.783 | 0.20 (0.25)* |
| Coker Gasoline (GR) | 4.76 | 1.42 (1.52)* |
| SHU product (GR) | 0.564 | 0.16 (0.18)* |
| Coker Gasoline (PR) | 3.2 | 0.95% (1.0)* |
| MRU Product ( PR)-1 | 0.78 | 0.22%(0.25)* |
| MRU Product (PR) -2 | 0.38 | 0.10%(0.12)* |
| MRU Product ( PR) - 3 | 0.44 | 0.12%(0.14)* |
| MRU Product ( PR) - 4 | 0.188 | 0.05%(0.06)* |

| | | |
|---|---|---|
| ***represents the diene concentration calculated as per UOP method** | | |

Table 1 gives a comparison of the Diene Value results obtained by UOP-326 method and the Diene Concentration determined by new GC-MS method. The Diene Value can be converted into Diene Concentration in the formula given in UOP-326 method. Diene Value and Diene Concentration of two different parameters but they can be correlated.

**Table 2:**

| **Results showing the diene content of a typical feed and product:** | | | |
|---|---|---|---|
| **S.No.** | **Diene** | **Coker Gasoline Feed (wt%)** | **SHU PRD (Selective Hydrogenation Unit-Product)** |
| 1. | 2,4-Pentadiene | 0.032 | ND |
| 2. | 1,3-Pentadiene (Z) | 0.077 | 0.011 |
| 3. | 1,3-Pentadiene (E) | 0.051 | 0.009 |
| 4. | 3-Me-1,3-Pentadiene (E) | 0.011 | ND |
| 5. | 3-Me-1,3-Pentadiene (Z) | 0.0035 | ND |
| 6. | 2,4-Hexadiene (E,E) | 0.0074 | 0.002 |
| 7. | 2,4-Hexadiene (Z,Z) | 0.010 | ND |
| 8. | 2,4-Hexadiene (E,Z) | 0.0001 | ND |
| 9. | 2,4-Hexadiene (Z,E) | 0.046 | 0.027 |
| 10. | 1,3-Hexadiene (E) | 0.002 | 0.0008 |
| 11. | 1,3-Hexadiene (Z) | 0.0366 | 0.002 |
| 12. | 1-Me-1,3-Cyclopentadiene(E) | 0.091 | 0.014 |
| 13. | 1-Me-1,3-Cyclopentadiene (Z) | 0.042 | ND |
| 14. | 2-Me-1,3-Pentadiene | 0.013 | 0.009 |
| 15. | 4-Me-1,3-Pentadiene | 0.025 | ND |
| 16. | 2,3-DM-1,3-Pentadiene | 0.0412 | 0.067 |
| 17. | 3,3-DM-1,3-Pentadiene | 0.0017 | ND |
| 18. | 5,5-DM-1,3-hexadiene | 0.162 | 0.018 |
| 19. | 1,3,5-heptatriene | 0.049 | ND |
| 20. | 2,3,4-TM-1,3-pentadiene | 0.007 | ND |
| 21. | 1-Me-cyclohexa-1,3-diene | 0.013 | ND |
| 22. | 2-Me-cyclohexa-1,3-diene | 0.035 | ND |
| | **Total Conjugated Dienes** | **0.75%** | **0.16%** |
| | **Diene Value (UOP-326)** | **3.4** | **0.752** |

Table 2 illustrates the change in the total diene concentration after selective hydrogenation process. The total diene content decreases in the process.

## Claims

1. A method of quantification of conjugated dienes in a feed or a product stream by a Gas Chromatography (GC), wherein the Gas Chromatography is coupled with Mass Spectrometry in a selective hydrogenation process (SHP), wherein said method comprises of:
a) passing a portion of a feed or a product stream to be processed by the SHP, through a GC column having a length ranging from 140 to 170 m;
b) determining concentration of each conjugated dienes by a mass spectrometer;
c) performing the SHP on the feed or the product stream to obtain a SHP product;
d) passing a portion of the SHP product through the GC column having a length ranging from 140 to 170 m;
e) determining concentration of each conjugated dienes by the mass spectrometer; and
f) comparing the concentration of the conjugated dienes of step (b) and (e) to determine the amount of hydrogenation of conjugated dienes in the SHP.

2. The method as claimed in claim 1, wherein the GC column length is 150 m.

3. The method as claimed in any of the preceding claim, wherein the GC column is a non-polar column and is operated in a temperature range of 60°C to 360°C.

4. The method as claimed in any of the claims 1 to 3, wherein the feed or the product stream is gasoline.

5. The method as claimed in any of the claims 1 to 3, wherein the conjugated dienes contain carbon in the range of Cs to Cs and are selected from cyclic, open chain, branched chain, or a mixture thereof.

6. The method as claimed in any of the claims 1 to 3, wherein the mass spectrometer is employed with a quadrupole mass detector wherein the mass detector is tuned for unit resolution in mass range of 1 to 1000 Da by using per-fluoro tertiary butyl amine as reference for calibration of masses.

7. The method as claimed in claim 6, wherein the mass detector is operated at a vacuum of 10⁻⁶ bar and temperature of filament and transfer line is 240-280°C.

8. The method as claimed in any of the preceding claims, wherein the method is used to separate and identify and estimates up to 25 different conjugated dienes selected from the group comprising of 2,4-pentadiene, 1,3-pentadiene (Z), 1,3-pentadiene (E), 2-methyl-1,3-pentadine (E), 2-methyl-1,3-pentadiene (Z), 3-methyl-1,3-pentadiene (E), 3-methyl-1,3-pentadiene (Z), 2,4-hexadiene (E,E), 2,4-hexadiene (Z,Z), 2,4-hexadiene (E,Z), 2,4-hexadiene (Z,E), 1,3-hexadiene (E), 1,3-hexadiene (Z), 1-methyl-1,3-cyclopentadiene (E), 1-methyl-1,3-cyclopentadiene (Z), 2-methyl-1,3-pentadiene, 4-methyl-1,3-pentadiene, 2-3-dimethyl-1,3-pentadiene, 3,3-dimethyl-1,3-pentadiene, 5-5-dimethyl-1,3-hexadiene, 1,3,5-heptatriene, 2,3,4-trimethyl-1,3-pentadiene, 1-methyl-cyclohexa-1,3-diene, 2-methyl-cyclohexa-1,3-diene and 1,3,5-trimethyl-1,3-pentadiene.

9. The method of claim 1, comprising quantification of conjugated dienes in a SHU feed and a product sample, wherein said method comprises of:
a) setting up the GC with the mass spectrometer (MS) with a 150 meter column non-polar column having dimensions 150 m x 0.23mm x 0.5mm;
b) tuning mass spectrometer for unit resolution in a mass range of 1 to 1000 Da using Per-fluoro tertiary butyl amine (PFTBA) as a reference for calibration of masses;
c) maintaining mass spectrometer (MS) at a high vacuum of 10⁻⁶ bar and the temperature of the filament and transfer line at 260°C;
d) setting up an oven program for separation of conjugated dienes in a SHU feed and a product matrix;
e) injecting a chloroform solution of a standard ; wherein the standard comprises of t-1,3-pentadiene, 3-methyl-1,3-pentadiene, 2, 4-dimethyl-1,3-pentadiene, 1,3-hexadiene, 2,4-hexadiene, 1,3-cyclohexadiene, 1,3-cyclooctadiene;
f) calculating response factor of the standard of step (e) and calculating an average response factor and selecting one conjugated diene as a representative diene for calibration;
g) preparing a calibration curve in the concentration range 200 ppm to 1% (weight/weight) against an internal standard; wherein the internal standard is dodecane;
h) analyzing actual SHU feed and product samples containing the internal standard and determining the peak area of conjugated dienes identified with the help of NIST mass spectral library; and
i) calculating the concentration of total conjugated dienes and estimating the concentration against calibration curve of the conjugated diene of step (f).

10. The method as claimed in claim 9, wherein representative diene for calibration is 1,3-cyclodiene.

## Patentansprüche

1. Verfahren zur Quantifizierung von konjugierten Dienen in einem Feed- oder Produktstrom durch eine Gaschromatographie (GC), wobei die Gaschromatographie mit Massenspektrometrie in einem selektiven Hydrierungsprozess (SHP) gekoppelt ist, wobei das Verfahren Folgendes enthält:
a) Durchleiten eines Teils eines Feed- oder Produktstroms, der durch den SHP verarbeitet werden soll, durch eine GC-Säule mit einer Länge im Bereich von 140 bis 170 m;
b) Bestimmung der Konzentration jedes konjugierten Diens durch ein Massenspektrometer;
c) Durchführen des SHP in dem Feed- oder Produktstrom, um ein SHP-Produkt zu erhalten;
d) Durchleiten eines Teils des SHP-Produkts durch die GC-Säule mit einer Länge im Bereich von 140 bis 170 m;
e) Bestimmen der Konzentration der einzelnen konjugierten Diene durch das Massenspektrometer; und
f) Vergleichen der Konzentration der konjugierten Diene aus Schritt (b) und (e), um die Menge der Hydrierung der konjugierten Diene im SHP zu bestimmen.

2. Verfahren nach Anspruch 1, wobei die Länge der GC-Säule 150 m beträgt.

3. Verfahren nach einem der vorhergehenden Ansprüche, wobei die GC-Säule eine unpolare Säule ist und in einem Temperaturbereich von 60°C bis 360°C betrieben wird.

4. Verfahren nach einem der Ansprüche 1 bis 3, wobei der Feed- oder Produktstrom aus Benzin besteht.

5. Verfahren nach einem der Ansprüche 1 bis 3, wobei die konjugierten Diene Kohlenstoff im Bereich von C₅ bis C₈ enthalten und aus zyklischem, offenkettigem, verzweigtkettigem oder einer Mischung davon ausgewählt werden.

6. Verfahren nach einem der Ansprüche 1 bis 3, wobei das Massenspektrometer mit einem Quadrupol-Massendetektor verwendet wird, wobei der Massendetektor auf eine Einheitsauflösung im Massenbereich von 1 bis 1000 Da abgestimmt wird, indem per-Fluortertiäres Butylamin als Referenz für die Kalibrierung der Massen verwendet wird.

7. Verfahren nach Anspruch 6, wobei der Massendetektor bei einem Vakuum von 10⁻⁶ Bar betrieben wird und die Temperatur des Filaments und der Transferleitung 240-280°C beträgt.

8. Verfahren nach einem der vorhergehenden Ansprüche, wobei das Verfahren verwendet wird, um auszusondern und zu kennzeichnen, und das bis zu 25 verschiedene konjugierte Diene einschätzt, die aus der Gruppe ausgewählt werden, die aus 2,4-Pentadien, 1,3-Pentadien (Z), 1,3-Pentadien (E) 2-Methyl-1,3-Pentadien (E), 2-Methyl-1,3-Pentadien (Z), 3-Methyl-1,3-Pentadien (E), 3-Methyl-1,3-Pentadien (Z), 2,4-Hexadien (E,E), 2,4-Hexadien (Z,Z), 2, 4-Hexadien (E,Z), 2,4-Hexadien (Z,E), 1,3-Hexadien (E), 1,3-Hexadien (Z), 1-Methyl-1,3-Cyclopentadien (E), 1-Methyl-1,3-Cyclopentadien (Z), 2-Methyl-1,3-Pentadien, 4-Methyl-1,3-Pentadien, 2-3-Dimethyl-1,3-Pentadien, 3,3-Dimethyl-1,3-Pentadien, 5-5-Dimethyl-1,3-Hexadien, 1,3,5-Heptatrien, 2,3,4-Trimethyl-1,3-Pentadien, 1
Methyl-Cyclohexa-1,3-Dien, 2-Methyl-Cyclohexa-1,3-Dien und 1,3,5-Trimethyl-1,3-Pentadien besteht.

9. Verfahren nach Anspruch 1, das die Quantifizierung von konjugierten Dienen in einem SHU Feed- und Produktmuster umfasst, wobei das Verfahren Folgendes aufweist:
a) Einrichten der GC mit dem Massenspektrometer (MS) mit einer unpolaren 150 m Säule mit den Abmessungen 150 m x 0,23 mm x 0,5 mm;
b) Abstimmen des Massenspektrometers für eine Einheitsauflösung in einem Massenbereich von 1 bis 1000 Da unter Verwendung von per-Fluor-tertiärem Butylamin (PFTB A) als Referenz für die Kalibrierung der Massen;
c) Halten des Massenspektrometers (MS) bei einem Hochvakuum von 10⁻⁶ Bar und der Temperatur des Filaments und der Transferleitung unter 260°C;
d) Einrichten eines Ofenprogramms für die Trennung von konjugierten Dienen in einem SHU-Feed und einer Produktmatrix;
e) Einspritzen einer Chloroformlösung eines Standards; wobei der Standard t-1,3-Pentadien, 3-Methyl-1,3-Pentadien, 2,4-Dimethyl-1,3-Pentadien, 1,3-Hexadien, 2,4-Hexadien, 1,3-Cyclohexadien, 1,3-Cyclooctadien umfasst;
f) Berechnen des Reaktionsfaktors des Standards aus Schritt (e) und Berechnen eines durchschnittlichen Reaktionsfaktors und Auswählen eines konjugierten Diens als repräsentatives Diens für die Kalibrierung;
g) Erstellen einer Kalibrierkurve im Konzentrationsbereich von 200 ppm bis 1 % (Gewicht/Gewicht) gegen einen internen Standard, wobei der interne Standard Dodekan ist;
h) Analyse der tatsächlichen SHU Feed- und Produktmuster, die den internen Standard enthalten, und Bestimmung der Peakfläche der konjugierten Diene, die mit Hilfe der NIST-Massenspektralbibliothek identifiziert wurden; und
i) Berechnen der Konzentration der gesamten konjugierten Diene und Einschätzen der Konzentration gegen die Kalibrierungskurve des konjugierten Diens aus Schritt (f).

10. Verfahren nach Anspruch 9, wobei das repräsentative Dien für die Kalibrierung 1,3-Cyclodien ist.

## Revendications

1. Procédé de quantification de diènes conjugués dans une alimentation ou un flux de produit par une chromatographie en phase gazeuse (GC), dans lequel la chromatographie en phase gazeuse est couplée à la spectrométrie de masse dans un procédé d'hydrogénation sélective (SHP), dans lequel ledit procédé comprend les étapes suivantes :
a) le passage d'une partie d'une alimentation ou d'un flux de produit à traiter par le SHP, à travers une colonne GC ayant une longueur comprise entre 140 et 170 m ;
b) la détermination de la concentration de chaque diène conjugué par un spectromètre de masse ;
c) la réalisation du SHP sur l'alimentation ou le flux de produit pour obtenir un produit SHP ;
d) le passage d'une partie du produit SHP à travers la colonne GC ayant une longueur allant de 140 à 170 m ;
e) la détermination de la concentration de chaque diène conjugué par le spectromètre de masse ; et
f) la comparaison de la concentration des diènes conjugués de l'étape (b) et (e), la détermination de la quantité d'hydrogénation des diènes conjugués dans le SHP.

2. Procédé selon la revendication 1, dans lequel la longueur de la colonne GC est de 150 m.

3. Procédé selon l'une quelconque des revendications précédentes, dans lequel la colonne GC est une colonne non polaire et fonctionne dans une plage de température de 60°C à 360°C.

4. Procédé selon l'une quelconque des revendications 1 à 3, dans lequel l'alimentation ou le flux de produit est de l'essence.

5. Procédé selon l'une quelconque des revendications 1 à 3, dans lequel les diènes conjugués contiennent du carbone dans la gamme de Cs à C₈ et sont choisis parmi les diènes cycliques, à chaîne ouverte, à chaîne ramifiée, ou un mélange de ceux-ci.

6. Procédé selon l'une quelconque des revendications 1 à 3, dans lequel le spectromètre de masse est utilisé avec un détecteur de masse quadripolaire, dans lequel le détecteur de masse est accordé pour une résolution unitaire dans la plage de masse de 1 à 1000 Da en utilisant la per-fluoro-butylamine tertiaire comme référence pour l'étalonnage des masses.

7. Procédé selon la revendication 6, dans lequel le détecteur de masse fonctionne sous un vide de 10⁻⁶ bar et la température du filament et de la ligne de transfert est de 240-280°C.

8. Procédé selon l'une quelconque des revendications précédentes, dans lequel le procédé est utilisé pour séparer et identifier et estimer jusqu'à 25 diènes conjugués différents choisis dans le groupe comprenant le 2,4-pentadiène, le 1,3-pentadiène (Z), le 1,3-pentadiène (E), le 2- méthyl-1,3-pentadiène (E), le 2-méthyl-1,3-pentadiène (Z), le 3-méthyl-1,3-pentadiène (E), le 3-méthyl-1,3-pentadiène (Z), le 2,4-hexadiène (E,E), le 2,4-hexadiène (Z,Z), le 2, 4-hexadiène (E,Z), le 2,4-hexadiène (Z,E), le 1,3-hexadiène (E), 1,3-hexadiène (Z), le 1-méthyl-1,3-cyclopentadiène (E), le 1-méthyl-1,3-cyclopentadiène (Z), le 2-méthyl-1,3-pentadiène, le 4- méthyl-1,3-pentadiène, le 2-3-diméthyl-1,3-pentadiène, le 3,3-diméthyl-1,3-pentadiène, le 5- 5-diméthyl-1,3-hexadiène, le 1,3,5-heptatriène, le 2,3,4-triméthyl-1,3-pentadiène, le 1-méthyl-cyclohexa-1,3-diène, le 2-méthyl-cyclohexa-1,3-diène et le 1,3,5-triméthyl-1,3-pentadiène.

9. Procédé de la revendication 1, comprenant la quantification des diènes conjugués dans une alimentation de SHU et un échantillon de produit, dans lequel ledit procédé comprend :
a) la mise en place de la GC avec le spectromètre de masse (SM) avec une colonne non polaire de 150 mètres ayant des dimensions de 150 m x 0,23 mm x 0,5 mm ;
b) l'accord du spectromètre de masse pour une résolution unitaire dans une gamme de masse de 1 à 1000 Da en utilisant la Per-fluoro tertiary butyl amine (PFTB A) comme référence pour l'étalonnage des masses ;
c) le maintien du spectromètre de masse (SM) à un vide poussé de 10⁻⁶ bar et la température du filament et de la ligne de transfert à 260°C ;
d) la mise en place d'un programme de four pour la séparation des diènes conjugués dans une alimentation de SHU et une matrice de produits ;
e) l'injection d'une solution de chloroforme d'un standard, dans lequel le standard comprend du t-1,3-pentadiène, du 3-méthyl-1,3-pentadiène, du 2,4-diméthyl-1,3-pentadiène, du 1,3-hexadiène, du 2,4-hexadiène, du 1,3-cyclohexadiène, du 1,3-cyclooctadiène ;
f) le calcul du facteur de réponse du standard de l'étape (e) et le calcul d'un facteur de réponse moyen et la sélection d'un diène conjugué comme diène représentatif pour l'étalonnage ;
g) la préparation d'une courbe d'étalonnage dans la plage de concentration de 200 ppm à 1% (poids/poids) par rapport à un standard interne ; dans laquelle le standard interne est le dodécane ;
h) l'analyse d'échantillons réels d'alimentation et de produit SHU contenant le standard interne et la détermination de la surface du pic des diènes conjugués identifiés à l'aide de la bibliothèque de spectres de masse du NIST ; et
i) le calcul de la concentration des diènes conjugués totaux et l'estimation de la concentration par rapport à la courbe d'étalonnage du diène conjugué de l'étape (f).

10. Procédé selon la revendication 9, dans lequel le diène représentatif pour l'étalonnage est le 1,3-cyclodiène.
